# FASCICULE DE BREVET EUROPEEN

(11) **EP 2 699 126 B1**
(45) Date de publication et mention de la délivrance du brevet: **16.05.2018**
(21) Numéro de dépôt: 11731349.4
(22) Date de dépôt: 12.07.2011
(51) Int. Cl.: A47C 31/00, A61G 7/05, A61B 5/00, A61B 5/11

(54) **DETECTEUR, MATELAS ET SYSTEME DE GESTION DE PARC DE MATELAS**
DETEKTOR, MATRATZE, UND SYSTEM ZUR VERWALTUNG EINES MATRATZENINVENTARS
DETECTOR, MATTRESS, AND SYSTEM FOR MANAGING A MATTRESS INVENTORY

(30) Priorité: 19.04.2011 EP 11163009
(43) Date de publication de la demande: 26.02.2014
(73) Titulaire: Elite SA, 1170 Aubonne (CH)
(72) Inventeur: Pugliese, François, CH-1806 St-Légier-La Chiésaz (CH); Harik, Louis, 1022 Chavannes-près-Renens (CH); Curty, Jari-Pascal, CH-1400 Yverson-les-Bains (CH)
(74) Mandataire: KATZAROV S.A.
(86) Numéro de dépôt international: PCT/EP2011/061857
(87) Numéro de publication internationale: WO 2012/143064

(56) Documents cités:
- JP-A- 2007 202 847
- DATABASE WPI Week 200561 Thomson Scientific, London, GB; AN 2005-594406 XP002662714, -& JP 2005 237977 A (MATSUSHITA DENKI SANGYO KK) 8 septembre 2005 (2005-09-08)

## Description

La présente invention concerne un détecteur pour déterminer et/ou mesurer l'utilisation d'un matelas. La présente invention concerne en particulier un détecteur destiné à être couplé à un matelas pour quantifier l'utilisation du matelas et ainsi déterminer par exemple son niveau d'usure, le coût de sa location, etc. La présente invention concerne également un matelas comprenant un tel détecteur et un système faisant usage des données récoltées à l'aide du ou des détecteurs.

Des matelas équipés de détecteurs sont connus de l'état de la technique. A titre d'exemple, JP2005237977 décrit un lit comprenant un matelas équipé d'un capteur de vibrations. Toutefois, les informations du capteur sont utilisées à d'autres fins, c'est-à-dire pour estimer la qualité du sommeil de l'utilisateur pour pouvoir le réveiller au cours d'une phase de sommeil favorable.

La gestion d'un parc de matelas, par exemple pour un établissement hôtelier, est généralement une tâche complexe et souvent coûteuse. En effet, le remplacement simultané de tous les matelas d'un établissement représente généralement un investissement important. D'autre part, il est difficile de déterminer l'usure de chaque matelas, certains ayant peut-être été utilisé plus souvent que d'autres. Il est donc difficile d'optimiser les coûts en ne remplaçant que les matelas qui en ont véritablement besoin.

Une solution pour permettre à un gérant d'établissement hôtelier de mieux planifier ses coûts serait par exemple de louer les matelas ou de les acheter par acomptes. Un désavantage d'une telle solution est cependant que les coûts liés à la location ou au remboursement des matelas sont fixes quel que soit le taux d'occupation de l'établissement, et que cela ne permet pas par ailleurs d'optimiser la gestion en ne remplaçant que les matelas usés.

Un but de la présente invention est donc de proposer un dispositif permettant de déterminer avec précision l'utilisation effective d'un ou de plusieurs matelas afin par exemple de pouvoir estimer au mieux le niveau d'usure et/ou le prix pour l'utilisation de chaque matelas.

Un autre but de la présente invention est de proposer un système permettant de gérer efficacement un parc de plusieurs matelas.

Ces buts et d'autres avantages sont atteints à l'aide d'un détecteur pour détecter l'utilisation d'un matelas, d'un matelas comprenant un tel détecteur, d'un système de détermination de l'utilisation de plusieurs matelas et d'un programme d'ordinateur pour implémenter un tel système, comprenant les caractéristiques des revendications indépendantes correspondantes.

Ces buts et d'autres avantages sont atteints à l'aide d'un détecteur pour déterminer l'utilisation d'un matelas, comprenant un capteur de mouvements pour détecter et/ou mesurer les mouvements du matelas, un capteur de niveau pour mesurer le niveau du matelas et/ou l'orientation du matelas.

Ces buts et d'autres avantages sont atteints également à l'aide d'un matelas comprenant un tel détecteur.

Ces buts et d'autres avantages sont atteints également à l'aide d'un système de gestion d'un parc de matelas comprenant une pluralité de tels matelas, un équipement distant en communication avec chacun des détecteurs de chaque matelas.

Ces buts et d'autres avantages sont atteints aussi à l'aide d'un programme informatique permettant de déterminer et/ou mesurer l'utilisation d'un matelas en fonction d'informations provenant d'un détecteur tel que décrit précédemment disposé dans le matelas, lorsque le programme est exécuté par un ordinateur.

Un détecteur comprenant un capteur de mouvements et un capteur de niveau permet de fournir suffisamment d'information sur l'état d'un matelas dans lequel est installé le détecteur pour pouvoir déterminer de manière fiable l'utilisation ou non du matelas, par exemple dans le cadre d'une nuitée d'hôtel. Le système de gestion permet alors de mesurer l'utilisation effective du matelas et de facturer la mise à disposition du matelas en fonction de cette utilisation effective.

La présente invention sera mieux comprise à la lecture de la description qui suit illustrée par les figures, où :
La figure 1 représente un matelas comprenant un détecteur selon une forme d'exécution ;
La figure 2 est une représentation schématique d'un détecteur selon une forme d'exécution ;
La figure 3 illustre un système de gestion d'un parc de plusieurs matelas selon une forme d'exécution.

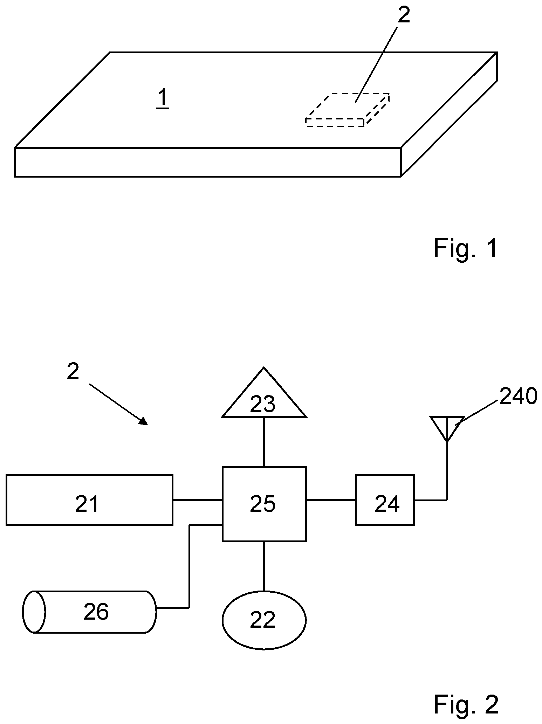

En référence à la figure 1, un matelas 1 comprend un détecteur 2 permettant de détecter et/ou de déterminer l'utilisation du matelas 1. Le détecteur 2 permet en particulier de déterminer si un utilisateur dort sur le matelas ou y a dormi. Selon une forme d'exécution, le détecteur 2 est disposé à l'intérieur du matelas 1, par exemple au cours de la fabrication du matelas. Le détecteur 2 est alors invisible et/ou inaccessible depuis l'extérieur du matelas 1. L'intégration du détecteur 2 dans le matelas 1 permet par exemple d'éviter toute manipulation involontaire et/ou non autorisée du détecteur 2. Elle permet également d'éviter tout endommagement du détecteur 2, par exemple lors du nettoyage, du retournement et/ou du déménagement du matelas.

Selon une autre forme d'exécution, le détecteur est fixé au matelas, de manière permanente ou amovible, après la fabrication du matelas. Le détecteur est alors par exemple attaché à l'extérieur du matelas, sur une de ses faces ou sur une tranche. Selon une variante, le détecteur est inséré à l'intérieur du matelas après la fabrication de ce dernier, par exemple au travers d'une ouverture pratiquée à cet effet ou prévue lors de la fabrication du matelas. De préférence, l'ouverture est ensuite refermée, par exemple recousue ou refermée à l'aide d'une fermeture éclair, de sorte que le détecteur soit invisible et inaccessible depuis l'extérieur du matelas.

Selon une forme d'exécution illustrée schématiquement à la figure 2, le détecteur 2 comprend un capteur de mouvements 21 pour détecter et/ou mesurer les mouvements d'un matelas auquel il est associé. Le capteur de mouvements 21 est par exemple un capteur de mouvements omnidirectionnel permettant de détecter et/ou mesurer les mouvements du matelas dans toutes les directions. Le capteur de mouvements 21 permet par exemple de détecter les mouvements d'un matelas comprenant le détecteur 2 lorsqu'un utilisateur s'assied ou se couche sur le lit correspondant.

Le capteur de mouvements 21 est par exemple un capteur électromécanique, par exemple un capteur de mouvements omnidirectionnel dont les caractéristiques électriques sont temporairement modifiées lorsqu'il est soumis à une accélération. Selon une forme d'exécution, le capteur de mouvements 21 comprend par exemple un interrupteur électrique qui est fermé lorsque le capteur de mouvements est immobile, en position de repos, quelle que soit l'orientation du capteur, et qui s'ouvre dès que le capteur est déplacé, quelle que soit la direction du mouvement du capteur. Lorsque le capteur est de nouveau au repos, l'interrupteur se referme.

Selon une variante, le capteur de mouvements est un MEMS comprenant par exemple un ou plusieurs accéléromètres orientés dans des directions différentes de manière à détecter toute accélération auquel le capteur de mouvements pourrait être soumis. Selon une autre variante, le capteur de mouvements 21 comprend un ou plusieurs capteurs de pression pour détecter et/ou mesurer la déformation de la structure du matelas due à ses mouvements au cours de son utilisation. D'autres formes de capteurs de mouvements, par exemple d'autres formes de capteurs de mouvements omnidirectionnels, sont cependant envisageables dans le cadre de l'invention. La combinaison de plusieurs capteurs de types différents est également possible dans le cadre de l'invention pour former le capteur de mouvements 21 du détecteur 2.

En référence à la figure 2, le détecteur 2 comprend en outre un capteur de niveau 22 pour mesurer le niveau et/ou l'orientation d'un matelas comprenant le détecteur 2. Le capteur de niveau 22 comprend par exemple une boussole, par exemple sous la forme d'un capteur de champs magnétiques, permettant de détecter et/ou mesurer de faibles champs magnétiques selon un ou plusieurs axes. Le capteur de niveau 22 permet ainsi par exemple de détecter les changements de niveau et/ou d'orientation du matelas auquel le détecteur 2 est associé et/ou de déterminer son orientation.

Optionnellement, le détecteur 2 comprend également un capteur de position 23 pour détecter par exemple des changements de position auxquels serait soumis un matelas comprenant le détecteur 2 et/ou pour déterminer la position absolue du matelas. Selon une forme d'exécution, le capteur de position 23 est un capteur de verticalité comprenant par exemple un interrupteur électrique qui est ouvert lorsque le matelas est posé sur une face et fermé lorsque le matelas est posé sur la face opposée. Le capteur de position 23 permet alors par exemple de déterminer sur quelle face est posé le matelas.

Le capteur de mouvements 21, le capteur de niveau 22 et le capteur de position 23 sont de préférence des composants électromécaniques de faible dimension pouvant être intégrés à un circuit électrique du détecteur 2, par exemple par leur soudage sur un circuit imprimé sur lequel sont formées des pistes conductrices électriquement pour mettre en contact entre eux les éléments du détecteur 2.

Le détecteur 2 comprend en outre des moyens de communication 24 pour communiquer avec un ou plusieurs équipements distants, non représentés à la figure 2, afin par exemple de transmettre des données relatives aux mesures et/ou détections effectuées par les capteurs du détecteur 2. Les moyens de communication 24 comprennent par exemple une interface de communication sans fil. Selon une forme d'exécution, les moyens de communication 24 comprennent par exemple une interface RFID, par exemple une interface RFID active. D'autres moyens de communication, filaires ou sans fil, sont cependant envisageables dans le cadre de l'invention. Ces moyens comprennent par exemple une interface Ethernet, USB, Bluetooth, WiFi, GSM ou de toute autre technologie adaptée.

Le détecteur 2 comprend par ailleurs un contrôleur 25, par exemple un microcontrôleur, pour commander les divers composants du détecteur 2. Le contrôleur 25 reçoit par exemple à intervalles réguliers ou en continu les données relatives aux mesures et/ou à l'état des capteurs de mouvements 21, de niveau 22 et/ou de position 23. Ces données sont alors traitées et/ou stockées par le contrôleur 25, par exemple dans une unité de mémoire non représentée, puis transmises, par exemple à intervalles régulier et/ou en continu aux moyens de communication 24 pour être transmis à un ou plusieurs équipements distants, par exemple au travers d'un réseau de télécommunication au moins partiellement sans fil.

Selon une forme d'exécution, le détecteur 2 comprend en outre une source d'énergie électrique 26, par exemple une pile ou une batterie, pour alimenter les composants du détecteur 2 en énergie électrique afin d'assurer leur fonctionnement. Les composants électromagnétiques du détecteur 2 sont de préférence des composants à faible consommation électrique et la capacité de la source d'énergie électrique 26 est dimensionnée de façon à permettre l'alimentation électrique du détecteur 2 durant au moins la durée de vie usuelle ou planifiée du matelas correspondant. Selon une variante, le détecteur est alimenté en énergie électrique depuis une source externe, par exemple au travers d'un branchement électrique filaire et/ou de moyens inductifs.

Selon une forme d'exécution, le détecteur 2 comprend un boîtier pour contenir au moins une partie des composants du détecteur 2. Le boîtier contient par exemple le circuit imprimé sur lequel sont fixés, par exemple soudés, et reliés électriquement entre eux, le capteur de mouvement 21, le capteur de niveau 22, le capteur de position 23, le contrôleur 25, la batterie 26 et au moins une partie des moyens de communication 24. Selon une forme d'exécution, les moyens de communication 24 comprennent une interface de communication sans fil avec une antenne 240 disposée à l'extérieur du boîtier, par exemple fixée à une paroi extérieure du boîtier.

Lors de l'association du détecteur 2 à un matelas, par exemple lors de l'installation du détecteur 2 à l'intérieur du matelas, le détecteur 2 est par exemple aligné selon une orientation précise par rapport au matelas. L'alignement relatif du détecteur 2 et du matelas étant alors connu, les informations provenant du détecteur 2, en particulier les informations de niveau et/ou d'orientation provenant du capteur de niveau 22 et/ou les informations de position provenant du capteur de position 23, permettent de déterminer le niveau et/ou l'orientation et/ou la position du matelas. Selon une variante, ou suite au référencement décrit plus haut, le matelas comprenant le détecteur 2 est soumis à une procédure de calibration afin de déterminer, respectivement de corriger, les données de l'alignement relatif du détecteur 2 et du matelas.

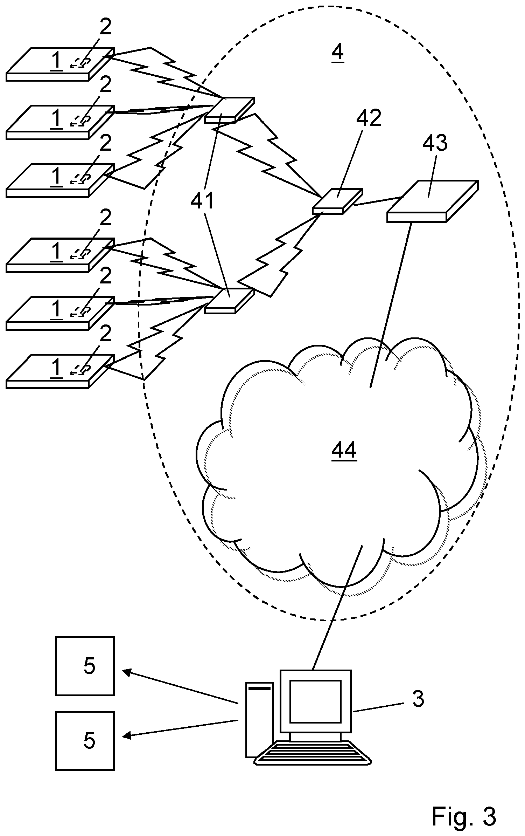

La figure 3 illustre schématiquement une forme d'exécution d'un système de gestion d'un parc de matelas, correspondant par exemple au parc de matelas de un ou plusieurs sites hôteliers, dans lequel chaque matelas 1 comprend un détecteur 2.

Le système comprend un équipement distant 3, par exemple un ordinateur, un serveur informatique, ou tout autre dispositif capable de recevoir, enregistrer et/ou traiter des informations sous forme de données numériques. Les matelas 1 sont en communication directe ou indirecte, permanente ou temporaire avec l'équipement distant 3 à travers un réseau de communication 4, par exemple un réseau de communication au moins partiellement sans fil. Le réseau de communication 4 comprend par exemple un ou plusieurs équipements de communication de proximité 41 en communication directe avec un ou plusieurs matelas 1 du parc de matelas sous gestion. L'équipement de communication de proximité 41 est un récepteur/émetteur par exemple sans fil utilisant un protocole de communication compatible avec celui utilisé par les moyens de communication des détecteurs 2. L'équipement de communication de proximité 41 comprend ainsi par exemple un récepteur et/ou émetteur RFID, Ethernet, USB, Bluetooth, WiFi, GSM ou de toute autre technologie adaptée.

Dans un site hôtelier, plusieurs équipements de communication de proximité 41 sont par exemple répartis dans l'hôtel de sorte que chaque matelas 1 du site soit en communication directe avec au moins un équipement de communication de proximité 41. Suivant le protocole et/ou la technologie utilisée et la configuration du site, l'hôtel est par exemple équipé d'un équipement de communication de proximité 41 par chambre, par couloir, par étage ou par bâtiment.

Optionnellement, le réseau de communication 4 comprend également un ou plusieurs relais 42 pour relayer les communications de et vers le ou les équipements de communication de proximité 41 si la portée de ces derniers est insuffisante. Les relais 42 sont par exemple des équipements de communication similaires, voire identiques, aux équipements de communication de proximité 41.

Selon une forme d'exécution, par exemple si l'équipement distant 3 est physiquement éloigné du ou des sites hôteliers sous gestion, le réseau de communication 4 comprend également une ou plusieurs interfaces de télécommunication 43, par exemple une antenne GSM, un point d'accès à l'Internet, un appareil téléphonique, etc., pour communiquer les données provenant des matelas 1 à travers les équipements de communication de proximité 41 et/ou les relais 42 à l'équipement distant 3. Selon cette forme d'exécution, le réseau de communication 4 comprend ainsi un réseau de télécommunication 44 privé ou public tel que par exemple un réseau téléphonique et/ou l'Internet. Suivant le réseau de télécommunication 44 et la technologie choisie, le réseau de communication 4 comprend par exemple une interface de télécommunication 43 par site hôtelier, bâtiment, quartier, etc.

La configuration et les équipements du réseau de communication 4 décrits ci-dessus sont donnés à titre d'exemple illustratif, mais en aucun cas limitatif. D'autres technologies et/ou configurations du réseau de communication 4 sont possible dans le cadre de l'invention. Le nombre et le type d'équipements et/ou de réseaux publics ou privés utilisés pour l'établissement de la communication mono- ou bidirectionnelle entre l'équipement distant 3 et les matelas 1 sous gestion peut en particulier varier selon les formes d'exécution.

Selon l'invention, des informations relatives à l'état et/ou aux mesures effectuées par le détecteur 2 de chaque matelas 1 sont transmises en continu ou à intervalles régulier à l'équipement distant 3 à travers le réseau de communication 4 sous la forme de données numériques. Ces informations sont ensuite stockées et/ou traitées par l'équipement distant 3 afin de déterminer l'usage qui est fait de chaque matelas 1 en fonction des mouvements, changements de direction, orientation et/ou niveau enregistrés par les capteurs du détecteur correspondant. Selon une forme d'exécution, l'équipement distant 3 détermine par exemple, sur la base des informations reçues de chaque détecteur 2, si le matelas 1 correspondant est ou a été utilisé dans le cadre d'une nuitée d'hôtel.

Selon une autre forme d'exécution, au moins une partie du stockage et/ou du traitement des informations provenant des détecteurs 2, en vue par exemple de la détermination du nombre de nuitées enregistrées par chaque matelas, est effectué par un équipement plus proche physiquement du parc de matelas que l'équipement distant 3, par exemple par le ou certains des relais 42 et/ou par un ou plusieurs équipements associés non représentés. Selon cette forme d'exécution, le ou les relais 42 et/ou les équipements associés comprennent un processeur et de la mémoire permettant de stocker et traiter les informations provenant des détecteurs 2, afin par exemple de déterminer le nombre de nuitées enregistrées pour chaque matelas 1 dans une période de temps donnée. Seul par exemple le nombre de nuitées par matelas 1 est alors transmis à l'équipement distant 3, par exemple à intervalles réguliers, par exemple une fois par jour, par semaine, ou par mois. Le traitement des informations des détecteurs 2 par un ou des relais 42 et/ou par un ou des équipements associés permet de limiter la quantité d'information transmise à l'équipement distant 3, ce qui est particulièrement avantageux, par exemple, en cas d'utilisation d'un réseau de télécommunication 44 publique dont l'utilisation est payante.

La répartition du stockage et du traitement des données provenant des détecteurs 2 est donnée ci-dessus à titre d'exemple pour deux formes d'exécution illustratives mais en aucun cas limitatives. En effet, la répartition des tâches de stockage et/ou de traitement peut être faite de diverses manières, en fonction par exemple de la répartition des capacités de stockage et/ou de calcul numériques du système, ainsi que du type et du coût des divers canaux de communication.

La détermination de l'utilisation d'un matelas 1 pour une nuitée se fait par exemple en premier lieu sur la base des informations provenant du capteur de mouvements 21, la détection de mouvements du matelas 1 sur une période prolongée étant un indice de son utilisation pour une nuitée. Les informations du capteur de mouvements 21 sont alors comparées avec les informations simultanées des autres capteurs du même détecteur 2 afin de déterminer avec une plus meilleure probabilité si les mouvements du matelas 1 détectés par le capteur de mouvements 21 correspondent effectivement à une nuitée et non par exemple à un déménagement, un retournement total ou partiel du matelas 1, un changement de draps, etc. Par exemple, la détection par le capteur de niveau 22 d'un changement de niveau et/ou d'orientation important du matelas 1 simultané aux mouvements détectés par le capteur de mouvements 21 pourrait être un indice que les mouvements du matelas 1 sont dus par exemple à un changement de draps durant lequel un coin du matelas a par exemple été soulevé, ou au déplacement du matelas 1. De manière similaire, si des changements de position sont détectés simultanément par le capteur de position 23, cela signifie probablement que les mouvements détectés sont dus à un retournement ou à un déménagement du matelas 1 plutôt qu'à une nuitée.

L'analyse des informations provenant des capteurs, en particulier du capteur de mouvement 21 et du capteur de niveau 22, permet également par exemple de déterminer si les mouvements enregistrés par le capteur de mouvements 21 sont dus à son utilisation pour une nuitée, c'est-à-dire aux mouvements d'une personne couchée sur le matelas, ou si ces mouvements sont dus à l'appui sur une partie seulement du matelas 1, par exemple à une personne s'asseyant sur le matelas 1 ou le dépôt d'un objet lourd tel qu'une valise. En effet, suivant l'emplacement du détecteur 2 dans le matelas 1, si le capteur de niveau 22 enregistre une forte inclinaison, il est probable que les mouvements soient dus à un appui local sur le matelas 1 plutôt qu'aux mouvements d'une personne allongée.

Outre la détermination des nuitées, les informations provenant du détecteur 2 permettent, selon une forme d'exécution, de déterminer le nombre et/ou la fréquence des retournements du matelas 1 à l'aide par exemple des informations provenant du capteur de position 23. La détermination du nombre et/ou de la fréquence des retournements du matelas 1 permet par exemple de déterminer à tout moment sur quelle face se trouve le matelas 1, afin par exemple, en combinaison avec les informations de nuitées, de déterminer combien de nuitées ont été effectuées sur chaque côté du matelas 1, et/ou dans un but de maintenance, les matelas devant par exemple être retournés à intervalles réguliers, par exemple en fonction des saisons. Selon une variante, le capteur de position 23 est un capteur de verticalité et les informations qui en proviennent permettent de déterminer directement sur quelle face est posé le matelas, le capteur de verticalité émettant un signal électrique déterminé lorsque le matelas 1 se trouve sur une face, et un signal électrique différent lorsque le matelas 1 se trouve sur la face opposée.

Les informations provenant du détecteur 2 permettent également, par exemple à l'aide des informations provenant du capteur de niveau 22 et/ou du capteur de position 23, de déterminer si le matelas 1 est en position horizontale, auquel cas il est probablement installé sur un lit et utilisé ou prêt à l'utilisation, ou si il est en position verticale, auquel cas il est probablement entreposé sur la tranche et donc inutilisable. S'il est déterminé que le matelas 1 est en position verticale, les informations provenant du capteur de mouvements 21 peuvent être alors ignorées dans le cadre de la détermination du nombre de nuitées.

Selon une variante, une partie du traitement des informations provenant des différents capteurs est effectuée dans le détecteur 2 lui-même afin par exemple de réduire la quantité de données transmises sur le réseau de communication 4 et/ou de réduire la charge de calcul pour l'équipement distant 3. Certaines conditions sont par exemple vérifiées par le contrôleur 25 afin de déterminer quelles informations seront transmises. Le contrôleur 25 est par exemple configuré pour ne pas transmettre d'information en provenance du capteur de mouvements 21 si les informations provenant du capteur de niveau 22 et/ou du capteur de position permettent de déterminer avec une probabilité supérieure à un certain seuil que le matelas est placé sur la tranche.

Selon une forme d'exécution, les informations transmises par les détecteurs 2 sont récoltées à distance et traitées par une entité indépendante de l'établissement hôtelier, par exemple par un fournisseur de matelas 1, qui gère alors par exemple l'équipement distant 3. L'utilisation de chaque matelas 1, en particulier le nombre de nuitées pour chaque matelas 1, est alors par exemple facturée périodiquement à un ou plusieurs utilisateurs 5 du ou des parcs de matelas 1 sous gestion, par exemple au(x) propriétaire(s) du ou des sites hôtelier correspondants. Les coûts facturés pour la mise à disposition des matelas 1 correspondent ainsi à leur utilisation réelle. D'autre part, la détermination du nombre de nuitées effectivement supportées par chaque matelas 1 permet de déterminer le niveau d'usure de chaque matelas 1 et d'optimiser la maintenance du parc de matelas par l'utilisateur 5 et/ou par le gestionnaire du ou des parcs de matelas 1, par exemple en ne remplaçant que les matelas 1 dont l'usure le justifie et/ou en organisant par exemple des rotations de matelas 1 entre les chambres du ou des sites hôteliers en fonction du taux d'occupation de chaque chambre.

De préférence, la réception, le stockage et/ou le traitement des informations provenant du ou des détecteurs 2 sont effectués automatiquement à l'aide d'un programme d'ordinateur exécuté par exemple au moins en partie sur l'équipement distant 3 et/ou sur tout autre équipement du système approprié, géré par exemple par le fournisseur de matelas. Selon une variante, le programme de gestion permet également de générer automatiquement les factures et/ou les rapports d'occupation de chaque matelas 1 à l'attention du ou des utilisateurs 5, par exemple des sites hôteliers. Selon une forme d'exécution, le programme permet en outre de gérer simultanément plusieurs parcs de matelas 1.

## Revendications

1. Détecteur (2) pour déterminer l'utilisation d'un matelas (1), comprenant :
- un capteur de mouvements (21) pour détecter et/ou mesurer les mouvements dudit matelas (1) ;
- un capteur de niveau (22) pour mesurer le niveau du matelas (1) et/ou l'orientation du matelas (1).

2. Détecteur (2) selon la revendication précédente, comprenant en outre un capteur de position (23) pour déterminer la position dudit matelas (1) et/ou pour détecter les changements de position dudit matelas (1).

3. Détecteur (2) selon l'une des revendications précédentes, ledit capteur de position (23) étant un capteur de verticalité.

4. Détecteur (2) selon l'une des revendications précédentes comprenant en outre des moyens de communication (24) pour communiquer avec l'équipement distant (3).

5. Détecteur (2) selon la revendication précédente, lesdits moyens de communication (24) étant des moyens de communication sans fil.

6. Matelas (1) comprenant un détecteur (2) selon l'une des revendications précédentes.

7. Matelas (1) selon la revendication précédente, ledit détecteur (2) étant disposé à l'intérieur dudit matelas (1).

8. Système de gestion d'un parc de matelas comprenant une pluralité de matelas (1) selon l'une des revendications 6 ou 7, un équipement distant (3) en communication avec chacun des détecteurs (2) de ladite pluralité de matelas (1).

9. Système selon la revendication précédente, comprenant en outre des relais (42) pour permettre la communication entre ledit équipement distant (3) et au moins une partie desdits détecteurs (2).

10. Système selon la revendication précédente, ledit équipement distant (3) étant configuré pour recevoir, stocker et/ou analyser des informations provenant de chacun des détecteurs (2) de ladite pluralité de matelas (1) et pour déterminer et/ou mesurer l'utilisation de chaque matelas (1) de ladite pluralité de matelas (1) en fonction desdites informations.

11. Programme informatique permettant de déterminer et/ou mesurer l'utilisation d'un matelas (1) en fonction d'informations provenant d'un détecteur (2) selon l'une des revendications 1 à 5 disposé dans ledit matelas (1) lorsque ledit programme est exécuté par un ordinateur.

12. Programme informatique selon la revendication précédente, permettant en outre de calculer un coût de location en fonction de ladite utilisation dudit matelas (1).

13. Programme informatique selon la revendication précédente, permettant en outre de déterminer un plan de rotation d'une pluralité de matelas (1) comprenant chacun un détecteur (2) selon l'une des revendications 1 à 5 en fonction de l'utilisation de chaque matelas (1) de ladite pluralité de matelas (1).

## Patentansprüche

1. Detektor (2) zum Bestimmen der Verwendung einer Matratze (1), umfassend:
- einen Bewegungssensor (21) zum Erfassen und/oder Messen der Bewegungen der besagten Matratze (1);
- einen Niveausensor (22) zum Messen der Lage der Matratze (1) und/oder der Orientierung der Matratze (1).

2. Detektor (2) gemäss dem vorhergehenden Anspruch, zudem umfassend einen Positionssensor (23) zum Bestimmen der Position der besagten Matratze (1) und/oder zum Erfassen von Änderungen der Position der besagten Matratze (1).

3. Detektor (2) gemäss einem der vorhergehenden Ansprüche, wobei der besagte Positionssensor (23) ein Vertikalitätssensor ist.

4. Detektor (2) gemäss einem der vorhergehenden Ansprüche, zudem umfassend Kommunikationsmittel (24) zum Kommunizieren mit der Femausrüstung (3).

5. Detektor (2) gemäss dem vorhergehenden Anspruch, wobei die besagten Kommunikationsmittel (24) drahtlose Kommunikationsmittel sind.

6. Matratze (1) mit einem Detektor (2) gemäss einem der vorhergehenden Ansprüche.

7. Matratze (1) gemäss dem vorhergehenden Anspruch, wobei der besagte Detektor (2) innerhalb der besagten Matratze (1) angeordnet ist.

8. Matratzeninventarverwaltungssystem mit einer Vielzahl von Matratzen (1) gemäss einem der Ansprüche 6 oder 7, wobei eine Femausrüstung (3) mit jedem der Detektoren (2) der besagten Mehrzahl von Matratzen (1) in Verbindung steht.

9. System gemäss dem vorhergehenden Anspruch, welches zudem Relais (42) umfasst, um die Kommunikation zwischen der besagten Femausrüstung (3) und mindestens einem Teil der Detektoren (2) zu ermöglichen.

10. System gemäss dem vorhergehenden Anspruch, wobei die besagte Femausrüstung (3) konfiguriert ist, um Informationen von jedem der Detektoren (2) der besagten Mehrzahl von Matratzen (1) zu empfangen, zu speichern und/oder zu analysieren, und um die Verwendung jeder Matratze (1) der besagten Mehrzahl von Matratzen (1) gemäss den besagten Informationen zu bestimmen und/oder zu messen.

11. Computerprogramm zum Bestimmen und/oder Messen der Verwendung einer Matratze (1) gemäss Informationen von einem Sensor (2) gemäss einem der Ansprüche 1 bis 5, der in der besagten Matratze (1) angeordnet ist, wenn das Programm von einem Computer ausgeführt wird.

12. Computerprogramm gemäss dem vorhergehenden Anspruch, welches zudem ermöglicht, Mietkosten gemäss der besagten Verwendung der besagten Matratze (1) zu berechnen.

13. Computerprogramm gemäss dem vorhergehenden Anspruch, welches es weiter erlaubt, einen Rotationsplan einer Vielzahl von Matratzen (1) zu bestimmen, die jeweils einen Detektor (2) gemäss einem der Ansprüche 1 bis 5 gemäss der Verwendung jeder Matratze (1) der besagten Vielzahl von Matratzen (1) umfassen.

## Claims

1. Detector (2) for determining the use of a mattress (1), comprising:
- a motion sensor (21) for detecting and/or measuring the movements of said mattress (1);
- a level sensor (22) for measuring the level of the mattress (1) and/or the orientation of the mattress (1).

2. Detector (2) according to the preceding claim, further comprising a position sensor (23) for determining the position of said mattress (1) and/or for detecting changes in position of said mattress (1).

3. Detector (2) according to one of the preceding claims, said position sensor (23) being a verticality sensor.

4. Detector (2) according to one of the preceding claims, further comprising communication means (24) for communicating with the remote equipment (3).

5. Detector (2) according to the preceding claim, said communication means (24) being wireless communication means.

6. Mattress (1) comprising a detector (2) according to one of the preceding claims.

7. Mattress (1) according to the preceding claim, said detector (2) being arranged inside said mattress (1).

8. Mattress inventory management system comprising a plurality of mattresses (1) according to one of claims 6 or 7, a remote equipment (3) in communication with each of the detectors (2) of said plurality of mattresses (1).

9. System according to the preceding claim, further comprising relays (42) to allow communication between said remote equipment (3) and at least a part of said detectors (2).

10. System according to the preceding claim, said remote equipment (3) being configured for receiving, storing and/or analysing information coming from each of the detectors (2) of said plurality of mattresses (1) and for determining and/or measuring the use each mattress (1) of said plurality of mattresses (1) according to said information.

11. Computer program for determining and/or measuring the use of a mattress (1) according to information coming from a sensor (2) according to one of claims 1 to 5 arranged in said mattress (1) when said program is run by a computer.

12. Computer program according to the preceding claim, further allowing a rental cost to be calculated according to said use of said mattress (1).

13. Computer program according to the preceding claim, further allowing a rotation plan of a plurality of mattresses (1) to be determined, each comprising a detector (2) according to one of claims 1 to 5 according to the use of each mattress (1) of said plurality of mattresses (1).
